# EUROPEAN PATENT SPECIFICATION

(11) **EP 0 905 239 B1**
(45) Date of publication and mention of the grant of the patent: **23.11.2005**
(21) Application number: 98307643.1
(22) Date of filing: 21.09.1998
(51) Int. Cl.: C12N 15/12, C07K 14/47, C12P 21/02, C07K 16/18

(54) **Gene encoding Afadin-1**
Gen kodierend für Afadin-1
Gène codant Afadin-1

(30) Priority: 22.09.1997 JP 25704397
(43) Date of publication of application: 31.03.1999
(73) Proprietor: Japan Science and Technology Agency, Kawaguchi-shi, Saitama 332-0012 (JP); JCR PHARMACEUTICALS Co., LTD., Ashiya-shi, Hyogo (JP)
(72) Inventor:
(74) Representative: Jones, Elizabeth Louise

(56) References cited:
- WO-A-94/26930
- KANO KYOKO ET AL: "AF-6, a putative target for Ras, and cell adhesion." CELL STRUCTURE AND FUNCTION, vol. 21, no. 6, 1996, page 641 XP000965752 Forty-ninth Annual Meeting of the Japan Society for Cell Biology;Kyoto, Japan; October 23-25, 1996 ISSN: 0386-7196
- PRASAD R ET AL: "Cloning of the ALL-1 fusion partner, the AF-6 gene, involved in acute myeloid leukemias with the t (6;11) chromosome translocation." CANCER RESEARCH, vol. 53, no. 23, 1993, pages 5624-5628, XP000960775 ISSN: 0008-5472
- DATABASE WPI Section Ch, Week 199740 Derwent Publications Ltd., London, GB; Class B04, AN 1997-429179 XP002154358 & JP 09 191879 A (KIRIN BREWERY KK), 29 July 1997 (1997-07-29)
- MANDAI KENJI ET AL: "Afadin: A novel actin filament-binding protein with one PDZ domain localized at cadherin-based cell-to-cell adherens junction." JOURNAL OF CELL BIOLOGY, vol. 139, no. 2, 1997, pages 517-528, XP002154356 ISSN: 0021-9525
- SAITO S. ET AL.: "Complete genomic structure DNA polymorphisms, and alernative splicing of the human AF-6 gene" DNA RESEARCH, vol. 5, 1998, pages 115-120, XP000972285
- IKEDA WATARU ET AL: "Afadin: A key molecule essential for structural organization of cell-cell junctions of polarized epithelia during embryogenesis." JOURNAL OF CELL BIOLOGY, vol. 146, no. 5, pages 1117-1131, XP000960772 ISSN: 0021-9525
- SAITO SUSUMU ET AL: "Definition of a commonly deleted region in ovarian cancers to a 300-kb segment of chromosome 6q27." CANCER RESEARCH, vol. 56, no. 24, 1996, pages 5586-5589, XP000960776 ISSN: 0008-5472
- BUCHERT MICHAEL ET AL: "The junction-associated protein AF-6 interacts and clusters with specific Eph receptor tyrosine kinases at specialized sites of cell-cell contact in the brain." JOURNAL OF CELL BIOLOGY, vol. 144, no. 2, 25 January 1999 (1999-01-25), pages 361-371, XP000907350 ISSN: 0021-9525

### SEQUENCE LISTING

(1) GENERAL INFORMATION:
   (i) APPLICANT:
      (A) NAME: Japan Science & Technology Corporation
      (B) STREET: 4-1-8 Hon-cho
      (C) CITY: Kawaguchi-shi
      (D) STATE: Saitama
      (E) COUNTRY: Japan
      (F) POSTAL CODE (ZIP):

      (A) NAME: Manabu WADA
      (B) STREET: 1-16 Momoyamadai
      (C) CITY: Tarumi-ku
      (D) STATE: Kobe-shi, Hyogo
      (E) COUNTRY: Japan
      (F) POSTAL CODE (ZIP):

      (A) NAME: Hiroshi OBAISHI
      (B) STREET: 6-1-7-205 Kitaochiai
      (C) CITY: Suma-ku
      (D) STATE: Kobe-shi, Hyogo
      (E) COUNTRY: Japan
      (F) POSTAL CODE (ZIP):
   (ii) TITLE OF INVENTION: Protein
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
   (vi) PRIOR APPLICATION DATA:
      (A) APPLICATION NUMBER: JP 257043/1997
      (B) FILING DATE: 22-SEP-1997
(2) INFORMATION FOR SEQ ID NO: 1:
   (i) SEQUENCE CHARACTERISTICS:
      (A) LENGTH: 1829 amino acids
      (B) TYPE: amino acid
      (C) STRANDEDNESS:
      (D) TOPOLOGY: linear
   (ii) MOLECULE TYPE: protein
   (vi) ORIGINAL SOURCE:
      (A) ORGANISM: Rat
      (F) TISSUE TYPE: fetal brain
   (xi) SEQUENCE DESCRIPTION: SEQ ID NO: 1:
   Met Ser Ala Gly Gly Arg Asp Glu Glu Arg Arg Lys Leu Ala Asp Ile
   1 5 10 15
   Ile His His Trp Asn Ala Asn Arg Leu Asp Leu Phe Glu Ile Ser Gln
   20 25 30
   Pro Thr Glu Asp Leu Glu Phe His Gly Val Met Arg Phe Tyr Phe Gln
   35 40 45
   Asp Lys Ala Ala Gly Asn Phe Ala Thr Lys Cys Ile Arg Val Ser Ser
   50 55 60
   Thr Ala Thr Thr Gln Asp Val Ile Glu Thr Leu Ala Glu Lys Phe Arg
   65 70 75 80
   Pro Asp Met Arg Met Leu Ser Ser Pro Lys Tyr Ser Leu Tyr Glu Val
   85 90 95
   His Val Ser Gly Glu Arg Arg Leu Asp Ile Asp Glu Lys Pro Leu Val
   100 105 110
   Val Gln Leu Asn Trp Asn Lys Asp Asp Arg Glu Gly Arg Phe Val Leu
   115 120 125
   Lys Asn Glu Asn Asp Ala Ile Pro Ala Lys Lys Ala Gln Ser Asn Gly
   130 135 140
   Pro Glu Lys Gln Glu Lys Glu Gly Val Ile Gln Asn Phe Lys Arg Thr
   145 150 155 160
   Leu Ser Lys Lys Glu Lys Lys Glu Lys Lys Lys Arg Glu Lys Glu Ala
   165 170 175
   Leu Arg Gln Ala Ser Asp Lys Glu Glu Arg Pro Ser Gln Gly Asp Asp
   180 185 190
   Ser Glu Asn Ser Arg Leu Ala Ala Glu Val Tyr Lys Asp Met Pro Glu
   195 200 205
   Thr Ser Phe Thr Arg Thr Ile Ser Asn Pro Glu Val Val Met Lys Arg
   210 215 220
   Arg Arg Gln Gln Lys Leu Glu Lys Arg Met Gln Glu Phe Arg Ser Ser
   225 230 235 240
   Asp Gly Arg Pro Asp Ser Gly Gly Thr Leu Arg Ile Tyr Ala Asp Ser
   245 250 255
   Leu Lys Pro Asn Ile Pro Tyr Lys Thr Ile Leu Leu Ser Thr Thr Asp
   260 265 270
   Pro Ala Asp Phe Ala Val Ala Glu Ser Leu Glu Lys Tyr Gly Leu Glu
   275 280 285
   Lys Glu Asn Pro Lys Asp Tyr Cys Ile Ala Arg Val Met Leu Pro Pro
   290 295 300
   Gly Ala Gln His Ser Asp Glu Arg Gly Ala Lys Glu Ile Ile Leu Asp
   305 310 315 320
   Asp Asp Glu Cys Pro Leu Gln Ile Phe Arg Glu Trp Pro Ser Asp Lys
   325 330 335
   Gly Ile Leu Val Phe Gln Leu Lys Arg Arg Pro Pro Asp Tyr Ile Pro
   340 345 350
   Lys Lys Met Lys Lys His Val Glu Gly Lys Pro Leu Lys Gly Lys Asp
   355 360 365
   Arg Ala Asp Gly Ser Gly Tyr Gly Ser Ala Leu Pro Pro Glu Lys Leu
   370 375 380
   Pro Tyr Leu Val Glu Leu Ser Pro Gly Arg Arg Asn His Phe Ala Tyr
   385 390 395 400
   Tyr Ser Tyr His Thr Tyr Glu Asp Gly Ser Asp Ser Arg Asp Lys Pro
   405 410 415
   Lys Leu Tyr Arg Leu Gln Leu Ser Val Thr Glu Val Gly Thr Glu Lys
   420 425 430
   Phe Asp Asp Asn Ser Ile Gln Leu Phe Gly Pro Gly Ile Gln Pro His
   435 440 445
   His Cys Asp Leu Thr Asn Met Asp Gly Val Val Thr Val Thr Pro Arg
   450 455 460
   Ser Met Asp Ala Glu Thr Tyr Val Asp Gly Gln Arg Ile Ser Glu Thr
   465 470 475 480
   Thr Met Leu Gln Ser Gly Met Arg Leu Gln Phe Gly Thr Ser His Val
   485 490 495
   Phe Lys Phe Val Asp Pro Ile Gln Asp His Val Leu Ser Lys Arg Ser
   500 505 510
   Val Asp Gly Gly Leu Met Val Lys Gly Pro Arg His Lys Pro Gly Ala
   515 520 525
   Val Gln Glu Thr Thr Phe Glu Leu Gly Gly Asp Ile His Ser Gly Thr
   530 535 540
   Ala Leu Pro Ala Ser Arg Ser Thr Thr Arg Leu Asp Ser Asp Arg Val
   545 550 555 560
   Ser Ser Ala Ser Ser Thr Ala Glu Arg Gly Met Val Lys Pro Met Ile
   565 570 575
   Arg Leu Asp Gln Glu Gln Asp Tyr Arg Arg Arg Glu Ser Arg Thr Gln
   580 585 590
   Asp Ala Ala Gly Pro Glu Leu Met Leu Pro Ala Ser Ile Glu Phe Arg
   595 600 605
   Glu Ser Ser Glu Asp Ser Phe Leu Ser Ala Ile Ile Asn Tyr Thr Asn
   610 615 620
   Ser Ser Thr Val His Phe Lys Leu Ser Pro Thr Tyr Val Leu Tyr Met
   625 630 635 640
   Ala Cys Arg Tyr Val Leu Ser Ser Gln His Arg Pro Asp Ile Ser Pro
   645 650 655
   Thr Glu Arg Thr His Lys Ala Ile Ala Val Val Asn Lys Met Val Ser
   660 665 670
   Met Met Glu Gly Val Ile Gln Glu Val Asp Gln Val Asp Gln Lys Gln
   675 680 685
   Lys Asn Ile Ala Gly Ala Leu Ala Phe Trp Met Ala Asn Ala Ser Glu
   690 695 700
   Leu Leu Asn Phe Ile Lys Gln Asp Arg Asp Leu Ser Arg Ile Thr Leu
   705 710 715 720
   Asp Ala Gln Asp Val Leu Ala His Leu Val Gln Met Ala Phe Lys Tyr
   725 730 735
   Leu Val His Cys Leu Gln Ser Glu Leu Asn Asn Tyr Met Pro Ala Phe
   740 745 750
   Leu Asp Asp Pro Glu Glu Asn Ser Leu Gln Arg Pro Lys Ile Asp Asp
   755 760 765
   Val Leu His Thr Leu Thr Gly Ala Met Ser Leu Leu Arg Arg Cys Arg
   770 775 780
   Val Asn Ala Ala Leu Thr Ile Gln Leu Phe Ser Gln Leu Phe His Phe
   785 790 795 800
   Ile Asn Met Trp Leu Phe Asn Arg Leu Val Thr Asp Pro Asp Ser Gly
   805 810 815
   Leu Cys Ser His Tyr Trp Gly Ala Ile Ile Arg Gln Gln Leu Gly His
   820 825 830
   Ile Glu Ala Trp Ala Glu Lys Gln Gly Leu Glu Leu Ala Ala Asp Cys
   835 840 845
   His Leu Ser Arg Ile Val Gln Ala Thr Thr Leu Leu Thr Met Asp Lys
   850 855 860
   Tyr Val Pro Asp Asp Ile Pro Asn Ile Asn Ser Thr Cys Phe Lys Leu
   865 870 875 880
   Asn Ser Leu Gln Leu Gln Ala Leu Leu Gln Asn Tyr His Cys Ala Pro
   885 890 895
   Asp Glu Pro Phe Ile Pro Thr Asp Leu Ile Glu Asn Val Val Ala Val
   900 905 910
   Ala Glu Asn Thr Ala Asp Glu Leu Ala Arg Ser Asp Gly Arg Asp Val
   915 920 925
   Gln Leu Glu Glu Asp Pro Asp Leu Gln Leu Pro Phe Leu Leu Pro Glu
   930 935 940
   Asp Gly Tyr Ser Cys Asp Val Val Arg Asn Ile Pro Asn Gly Leu Gln
   945 950 955 960
   Glu Phe Leu Asp Pro Leu Cys Gln Arg Gly Phe Cys Arg Leu Val Pro
   965 970 975
   His Thr Arg Ser Pro Gly Thr Trp Thr Ile Tyr Phe Glu Gly Ala Asp
   980 985 990
   Tyr Glu Ser His Leu Met Arg Glu Asn Thr Glu Leu Thr Gln Pro Leu
   995 1000 1005
   Arg Lys Glu Pro Glu Val Ile Thr Val Thr Leu Lys Lys Gln Asn Gly
   1010 1015 1020
   Met Gly Leu Ser Ile Val Ala Ala Lys Gly Ala Gly Gln Asp Lys Leu
   1025 1030 1035 1040
   Gly Ile Tyr Val Lys Ser Val Val Lys Gly Gly Ala Ala Asp Val Asp
   1045 1050 1055
   Gly Arg Leu Ala Ala Gly Asp Gln Leu Leu Ser Val Asp Gly Arg Ser
   1060 1065 1070
   Leu Val Gly Leu Ser Gln Glu Arg Ala Ala Glu Leu Met Thr Arg Thr
   1075 1080 1085
   Ser Ser Val Val Thr Leu Glu Val Ala Lys Gln Gly Ala Ile Tyr His
   1090 1095 1100
   Gly Leu Ala Thr Leu Leu Asn Gln Pro Ser Pro Met Met Gln Arg Ile
   1105 1110 1115 1120
   Ser Asp Arg Arg Gly Ser Gly Lys Pro Arg Pro Lys Ser Glu Gly Phe
   1125 1130 1135
   Glu Leu Tyr Asn Asn Ser Ala Gln Asn Gly Ser Pro Glu Ser Pro Gln
   1140 1145 1150
   Met Pro Trp Thr Glu Tyr Ser Glu Pro Lys Lys Leu Pro Gly Asp Asp
   1155 1160 1165
   Arg Leu Met Lys Asn Arg Ala Asp His Arg Ser Ser Pro Asn Val Ala
   1170 1175 1180
   Asn Gln Pro Pro Ser Pro Gly Gly Lys Ser Pro Tyr Thr Ser Gly Thr
   1185 1190 1195 1200
   Ala Ala Lys Ile Thr Ser Val Ser Thr Gly Asn Leu Cys Thr Glu Glu
   1205 1210 1215
   Gln Thr Pro Pro Pro Arg Pro Glu Ala Tyr Pro Ile Pro Thr Gln Thr
   1220 1225 1230
   Tyr Thr Arg Glu Tyr Phe Thr Phe Pro Ala Ser Lys Ser Gln Asp Arg
   1235 1240 1245
   Met Ala Pro Val Gln Asn Gln Trp Pro Asn Tyr Glu Glu Lys Pro His
   1250 1255 1260
   Met His Thr Glu Ser Asp His Ala Ser Ile Ala Ile Gln Arg Val Thr
   1265 1270 1275 1280
   Arg Ser Gln Glu Glu Leu Arg Glu Glu Lys Val Tyr Gln Leu Glu Arg
   1285 1290 1295
   His Arg Val Glu Ser Gly Met Asp Arg Lys Cys Asp Ser Asp Met Trp
   1300 1305 1310
   Ile Asn Gln Ser Ser Ser Val Glu Ser Ser Thr Ser Ser Gln Glu His
   1315 1320 1325
   Leu Asn His Ser Ser Lys Ser Val Thr Pro Ala Ser Thr Leu Thr Lys
   1330 1335 1340
   Ser Gly Pro Gly Arg Trp Lys Thr Pro Ala Ala Val Leu Pro Thr Pro
   1345 1350 1355 1360
   Val Ala Val Ser Gln Pro Ile Arg Thr Asp Leu Pro Pro Pro Pro Pro
   1365 1370 1375
   Pro Pro Pro Ala His Tyr Thr Ser Asp Phe Asp Gly Ile Ser Met Asp
   1380 1385 1390
   Leu Pro Leu Pro Pro Pro Pro Ala Asn Gln Ala Ala Pro Gln Ser Ala
   1395 1400 1405
   Gln Val Ala Ala Ala Glu Arg Lys Lys Arg Glu Glu His Gln Arg Trp
   1410 1415 1420
   Tyr Glu Lys Glu Lys Ala Arg Leu Glu Glu Glu Arg Glu Arg Lys Arg
   1425 1430 1435 1440
   Arg Glu Gln Glu Arg Lys Leu Gly Gln Met Arg Thr Gln Ser Leu Asn
   1445 1450 1455
   Pro Ala Ser Phe Ser Pro Leu Ala Thr Gln Ala Lys Pro Glu Lys Pro
   1460 1465 1470
   Ser Thr Leu Gln Arg Pro Gln Glu Thr Val Ile Arg Glu Leu Gln Pro
   1475 1480 1485
   Gln Gln Gln Pro Arg Thr Ile Glu Arg Arg Asp Leu Gln Tyr Ile Thr
   1490 1495 1500
   Ile Ser Lys Glu Glu Leu Ser Ser Gly Asp Ser Leu Ser Pro Asp Pro
   1505 1510 1515 1520
   Trp Lys Arg Asp Ala Arg Glu Lys Leu Glu Lys Gln Gln Gln Met His
   1525 1530 1535
   Ile Val Asp Met Leu Ser Lys Glu Ile His Glu Leu Gln Asn Lys Gly
   1540 1545 1550
   Asp Arg Thr Ala Glu Glu Ser Asp Arg Leu Arg Lys Leu Met Leu Glu
   1555 1560 1565
   Trp Gln Phe Gln Lys Arg Leu Gln Glu Ser Lys Gln Lys Asp Glu Asp
   1570 1575 1580
   Asp Asp Glu Glu Glu Asp Asp Asp Val Asp Thr Met Leu Ile Met Gln
   1585 1590 1595 1600
   Arg Leu Glu Ala Glu Arg Arg Ala Arg Leu Gln Asp Glu Glu Arg Arg
   1605 1610 1615
   Arg Gln Gln Gln Leu Glu Glu Met Arg Lys Arg Glu Val Glu Asp Arg
   1620 1625 1630
   Val Arg Gln Glu Glu Asp Gly Arg His Gln Glu Glu Glu Arg Val Lys
   1635 1640 1645
   Arg Asp Ala Glu Glu Lys Arg Arg Gln Glu Glu Gly Tyr Tyr Ser Arg
   1650 1655 1660
   Leu Glu Ala Glu Arg Arg Arg Gln His Glu Glu Ala Ala Arg Arg Leu
   1665 1670 1675 1680
   Leu Glu Pro Glu Glu Pro Gly Leu Ser Arg Pro Pro Leu Pro Gln Asp
   1685 1690 1695
   Tyr Glu Pro Pro Ser Gln Ser Ser Ala Pro Ser Ala Pro Pro Pro Pro
   1700 1705 1710
   Pro Gln Arg Asn Ala Ser Tyr Leu Lys Thr Gln Val Leu Ser Pro Asp
   1715 1720 1725
   Ser Leu Phe Thr Ala Lys Phe Val Ala Tyr Asp Asp Asp Asp Glu Glu
   1730 1735 1740
   Glu Asn Tyr Val Pro Ala Gly Pro Asn Ser Tyr Ser Gly Ser Ala Gly
   1745 1750 1755 1760
   Thr Thr Ala Gly Thr Tyr Asp Ala Pro Arg Asp Thr Arg Glu Lys Leu
   1765 1770 1775
   Ser Arg Ser Gln Asp Ala Asp Leu Pro Gly Ser Ser Gly Ala Pro Glu
   1780 1785 1790
   Asn Leu Thr Phe Arg Glu Arg Gln Arg Leu Phe Ser Gln Gly Gln Asp
   1795 1800 1805
   Val Ser Asp Lys Val Lys Ala Ser Arg Lys Leu Thr Glu Leu Glu Asn
   1810 1815 1820
   Glu Leu Asn Thr Lys
   1825

## Description

The present invention relates to a novel actin filament binding protein "1-Afadin". More precisely, the present invention relates to a novel animal protein 1-Afadin that contributes to the cell-to-cell adherens junction (AJ) which has an important role in individual formation of animals and pathogenesis.

In various cellular events, such as cell adhesion, cell motility, and cell shape determination, specialized membrane domains are formed with transmembrane proteins, such as cell adhesion molecules, receptors, and channels, and these domains are often associated with the actin cytoskeleton (Biochem. Biophys. Acta 737:305-341, 1983; Curr. Opin. Cell Biol. 1:103-109, 1989; Cell Motil. Cytoskeleton 20:1-6, 1991; Curr. Opin. Cell Biol. 3:849-853, 1991; Science 258:955-964, 1992; Curr. Opin. Cell Biol. 4:834-839, 1992; Curr. Opin. Cell Biol. 5:653-660, 1993; Trends Biochem. Sci. 22: 53-58, 1997). The linkage between the actin cytoskeleton and the plasma membrane plays a crucial role in these cellular events, and proteins linking the actin cytoskeleton to the transmembrane proteins have been identified. However, the molecular basis of the linkage between the actin cytoskeleton and the plasma membrane is not fully understood.

To understand this molecular linkage, the cell adhesion sites have been studied extensively (Biochem. Biophys. Acta 737:305-341, 1983; Curr. Opin. Cell Biol. 1:103-109, 1989; Cell Motil. Cytoskeleton 20:1-6, 1991; Curr. Opin. Cell Biol. 3:849-853, 1991; Science 258:955-964, 1992; Curr. Opin. Cell Biol. 4:834-839, 1992; Curr. Opin. Cell Biol. 5:653-660, 1993; Trends Biochem. Sci. 22:53-58, 1997). As a result, the actin filament (F-actin)-associated cell adhesion sites are subclassed into two types: cell-to-cell and cell-to-matrix adherens junctions. Many linker proteins have been identified at the cell-to-cell AJ where cadherins interact with each other at the extracellular surface (Development 102:639-655, 1988; Cell Motil. Cytoskeleton 20:1-6, 1991: Science 251: 1451-1455, 1991; Curr. Opin. Cell Biol. 4:834-839, 1992; EMBO J. 8:1711-1717, 1989; Cell 65:849-857, 1991; Science 251:1451-1455, 1991; Curr. Opin. Cell Biol. 4:834-839, 1992). Among these binding proteins, α-catenin interacts directly with F-actin (Proc. Natl. Acad. Sci. USA 92, 8813-8817, 1995). α-Catenin also interacts indirectly with F-actin through α-actinin and/or ZO-1 (J. Cell. Biol. 130:67-77, 1995; J. Cell. Biol. 138:181-192, 1997). Further, Vinculin, another F-actin-binding protein, is concentrated at the cell-to-cell AJ, although the molecules with which it interacts at the cell-to-cell AJ have not yet been identified (Cell Motil. Cytoskeleton 20:1-6, 1991; Curr. Opin. Cell Biol. 4:834-839, 1992). At cell-to-matrix AJ where integrin interacts with matrix proteins at the extracellular surface, the cytoplasmic domain directly or indirectly interacts with F-actin binding proteins, including α-actinin, vinculin, and talin (Ann. Rev. Cell Dev. Biol. 11:379-416, 1995).

As described above, many F-actin-binding proteins appear to serve as linkers of the actin cytoskeleton to the plasma membrane cadherin and integrin.

On the other hand, the linkage between the actin cytoskeleton and the plasma membrane is also important for neuron-specific events, such as growth cone pathfinding and subsequent formation and maintenance of synaptic junction (Neuron 1:761-772, 1988; Science 242:708-715, 1988; Curr. Opin. Neurobiol. 4:43-48, 1994; Curr. Opin. Neurobiol. 4:640-647, 1994; Cell 83:171-176, 1995). However, it remains to be clarified which molecules link the actin cytoskeleton to the plasma membrane in these neuron-specific events.

To address this issue, the inventors of the present patent application have isolated several novel F-actin-binding proteins from rat brain and analyzed the structure of proteins particularly specific to neural tissue and concentrated at the synapse. A patent application for this subject matter has already been filed (Japanese Patent Application No. 9-92615). The protein of the prior invention (hereinafter, referred to as "neurabin" according to the inventor's nomenclature) has one F-actin-binding domain and one PDZ domain. The PDZ domain has been found in a variety of proteins, some of which are localized at cell-to-cell junctions, such as PSD-95/SAP9O at synaptic junctions (Neuron 9:929-942, 1992; J. Biol. Chem. 268:4580-4583, 1993), Dlg at septate junctions (Cell 66:451-464, 1991), ZO-1 and ZO-2 at tight junctions (J. Cell Biol. 193:755-766, 1986; Proc. Natl. Acad. Sci. USA 88:3460-3464, 1991; J. Cell Biol. 121:491-502, 1993; J. Cell Biol. 123:1049-1053, 1993; Proc. Natl. Acad. Sci. USA 90:7834-7838, 1993; J. Cell Biol. 124:949-961, 1994). In addition, recent studies have revealed that the PDZ domain binds to unique C-terminal motifs of target proteins (Trends Biochem. Sci. 21:455-458, 1996), which are found in a large number of transmembrane proteins, such as the N-methyl-D-aspartate receptor and Shaker-type K⁺ channel (Nature 378:85-88, 1995; Science 269:1737-1740, 1995; J. Neurosci. 16:2157-2163, 1996).

From the various findings described above, it is likely that neurabin, the protein of the prior invention found by the present inventors, serves as a linker of the actin cytoskeleton to a transmembrane protein(s) at synapses.

However, all aspects of the molecular basis for the cell-to-cell adhesion have not yet been clarified. Such clarification is necessary to identify further actin filament-binding proteins.

In addition, there is a possibility that these proteins will lead to clarification of, for example, the mechanisms of infiltration and metastasis of carcinoma, and it is expected that this will allow the development of diagnostic methods for determining the malignancy of carcinomas, therapeutic methods or agents for treating carcinomas and the like.

The present invention has been completed with the above in mind. An object of the present invention is to provide a novel actin filament-binding protein contributing to the cell-to-cell adhesion, simultaneously clarifying its structure (amino acid sequence) and its properties.

Another object of the present invention is to provide suitable material to allow genetic engineering manipulation of the actin filament-binding protein.

Thus, viewed from one aspect the present invention provides an actin filament-binding protein l-Afadin having the amino acid sequence of SEQ ID NO: 1. Corresponding proteins or peptides thereof, derived from any animal, having an amino acid sequence substantially the same as that of SEQ ID NO:1, especially from humans may be obtained, although they do not form part of the invention. As referred to herein substantially the same refers to proteins having for example 80% or more sequence identity, e.g. 85 or 90% identity to the amino acid sequence of SEQ ID NO:1. Peptide fragments (of 5 or more amino acid residues, e.g. more than 20 or more than 50 residues), e.g. antigenic fragments, can be obtained from proteins of the invention containing any partial amino acid sequence in the amino acid sequence of SEQ ID NO: l or from a sequence substantially similar thereto, e.g. derived from a different animal. These peptide fragments can be used as an antigen for producing antibodies. In addition, the protein can be fused with any other proteins (for example, fluorescent proteins and the like).

Functionally equivalent variants or precursors of the above defined proteins or peptide fragments may be obtained, although they do not form part of the present invention. "Functionally-equivalent" is used to define proteins or peptides related to or derived from the native protein, wherein the amino acid sequence has been modified by single or multiple amino acid substitution, addition (e.g the fusion proteins described above) and/or deletion and also sequences where the amino acids have been chemically modified, including by deglycosylation or glycosylation, but which nonetheless retain functional activity, ie which are capable of raising antibodies which bind to proteins of the invention and/or which exhibit similar F-actin binding properties to 1-Afadin described herein. Functionally-equivalent variants include natural biological variants (e.g. from different genera or species or allelic or geographical variants within a species) and derivatives prepared using known techniques.

The protein of the present invention can be isolated from human or other animal organs, cell lines and so on by the known methods, particularly by methods described herein and such method of preparing proteins of the invention, comprising the steps described in the Examples, form a further aspect of the invention. Proteins obtainable by such methods form a further aspect of the invention. When the protein is used in the form of a peptide, it can also be prepared by chemical synthesis based on the amino acid sequence provided by the present invention. Alternatively, it can be obtained by production in vitro using recombinant DNA techniques with a cDNA fragment provided by the present invention.

For example, when the protein is obtained by recombinant DNA techniques, nucleic acid fragments, such as a cDNA fragment can be inserted into an appropriate expression vector, and the protein of the present invention can be mass-expressed from the cells (such as Escherichia coli, Bacillus subtilis, yeast, animal cell and the like) which are transformed with the recombinant vector. Specifically, for example, when the protein is expressed in a microorganism such as E. coli, an expression vector is prepared by inserting the cDNA of the present invention into an expression vector having an origin which can be replicated in the microorganism, a promoter, a ribosome-binding site, cDNA cloning site, and a terminator. Host cells are transformed with the expression vector, and then the obtained transformant is cultured so that the protein encoded by the cDNA is mass-produced in the microorganism. Alternatively, the protein can be expressed as a fusion protein with another protein. The simple protein encoded by the cDNA can be obtained by cleaving the obtained fusion protein with an appropriate protease. On the other hand, when it is desired to express the protein of the present invention in animal cells, the cDNA fragment is inserted into an expression vector for animal cells having a promoter for animal cells, a splicing region, a poly (A)-addition site, and then the vector is introduced so that the protein of the present invention is expressed in the animal cells.

The above mentioned cloning and expression vectors containing nucleic acid molecules of the invention (as defined below), methods for preparing recombinant nucleic acid molecules according to the invention (comprising inserting nucleotide sequences encoding the protein into vector nucleic acid), transformed or transfected prokaryotic or eukaryotic host cells containing such vectors and the synthetic polypeptides expressed by these systems form further aspects of the invention.

In a further aspect, the present invention provides a genomic DNA sequence (or a nucleic acid molecule containing said sequence) which is a gene of a human or other animal which encodes the above protein, for example to which the cDNA or a partial sequence thereof is complementary or which hybridizes thereto.
Appropriate hybridizing conditions are described below. For example, it can be isolated from any genome library using a cDNA of the present invention or a partial sequence thereof as a probe.

In a yet further aspect the present invention provides a nucleic acid molecule comprising a nucleotide sequence encoding the proteins of the invention. Such nucleic acid molecules may be single or double stranded DNA, cDNA or RNA, preferably DNA, and include degenerate, substantially homologous and hybridising sequences which are capable of coding for the protein/polypeptide/peptide of the invention.

For example, the invention provides a cDNA molecule encoding a protein having the amino acid sequence of SEQ ID NO: 1. In reference to nucleic acid molecules, "substantially homologous" refers to sequences displaying at least 80%, preferably at least 85 or 90% sequence homology. Hybridizing sequences include within their scope those sequences binding under conditions of high stringency, e.g. 2XSSC, 65°C (where SSC = 0.15M NaCl, 0.015M sodium citrate, pH 7.2) as well as those which but for the degeneracy of the code would hybridize under the above-mentioned conditions.

A clone of the cDNA of the present invention can easily be obtained by screening a cDNA library prepared from rat by means of an oligonucleotide probe synthesized on the basis of the base sequence of the fragment. Alternatively, using the oligonucleotide as a primer, the desired cDNA can be synthesized by the polymerase chain reaction (PCR) method. Generally, polymorphism is frequently observed in animal genes as a result of individual variation. Therefore, it is to be appreciated that any cDNA having a single or plural addition, deletion and/or substitution of a nucleotide by other nucleotide is included in the present invention providing it encodes the protein of the invention. Proteins which have a single or plural addition, deletion and/or substitution of amino acid by another amino acid but has an activity of the protein having the amino acid sequence of SEQ ID NO: 1, ie. a functionally equivalent variant thereof may be obtained but are not covered by the present invention.

cDNA can encode a partial amino acid sequence, a ie. peptide fragment, which is a continuous sequence of 10 bps or more (e.g. oligonucleotides with 10-20 base pairs). DNA fragments (sense strand and antisense strand) comprising the continuous sequence can be used as probes for gene diagnosis.

Antibodies can be prepared using the actin filament-binding protein 1-Afadin as an immunogen. Antibodies include antigen-binding fragments of the antibodies (e.g. F(ab)², Fab and Fv fragments ie. fragments of the variable region of the antibody. Antibodies may be obtained as polyclonal antibodies or monoclonal antibodies by known methods using the above-described protein itself or a partial peptide (ie. fragment thereof) as the antigen.

The present invention will now be described by way of the following Examples, which should not be construed as a limitation upon the scope of the present invention, with reference to the Figures, in which:-
Figure 1 shows the results of Mono Q column chromatography: (a) absorption at 280 nm (A₂₈₀); (b) blot overlay with ¹²⁵I-labelled F-actin; and (c) protein staining after SDS-PAGE;
Figure 2 shows a schematic drawing of the cDNA of l-Afadin (p205) and that of s-Afadin (p190);
Figure 3 shows (a) the F-actin-binding activity of various fragments of recombinant l-Afadin: on the left is the results of the ¹²⁵I-labelled F-actin blot overlay; on the right is the result of Western blot analysis, and (b) schematic drawings of the structures of l-Afadin and s-Afadin;
Figure 4 shows results of analysis of tissue distribution of l-Afadin: (a) Northern blot analysis, and (b) Western blot analysis with (b1) anti-l-Afadin antibody and (b2) anti-1-Afadin/s-Afadin antibody;
Figure 5 shows the biochemical properties of l-Afadin including (a) the inhibition of F-actin binding activity of l-Afadin by myosin S1, (b) the increase in viscosity of F-actin by l-Afadin, and (c) the binding of His6-l-Afadin-C to F-actin;
Figure 6 shows photographs indicating the localization of l-Afadin, E-cadherin and vinculin in various rat and mouse tissues;
Figure 7 shows photographs indicating the localization of 1-Afadin and ZO-1 in EL cells;
Figure 8 shows photographs indicating the different localizations of 1-Afadin, ZO-1 and desmoplakin; and
Figure 9 shows photographs indicating the localization of l-Afadin in rat small intestine.

### Example 1: Identification and purification of the actin-binding protein 1-Afadin

Growth cones were isolated from rat fetal brain and subjected to the blot overlay method (Cell Motil. Cytoskeleton, 18:164-179, 1991) with ¹²⁵I-labelled F-actin to identify a band corresponding to a molecular weight of 205 kDa (p205). The result of the competition experiments showed that the protein bound specifically to F-actin but did not bind to G-actin (actin monomer), indicating that the protein was an F-actin-binding protein.

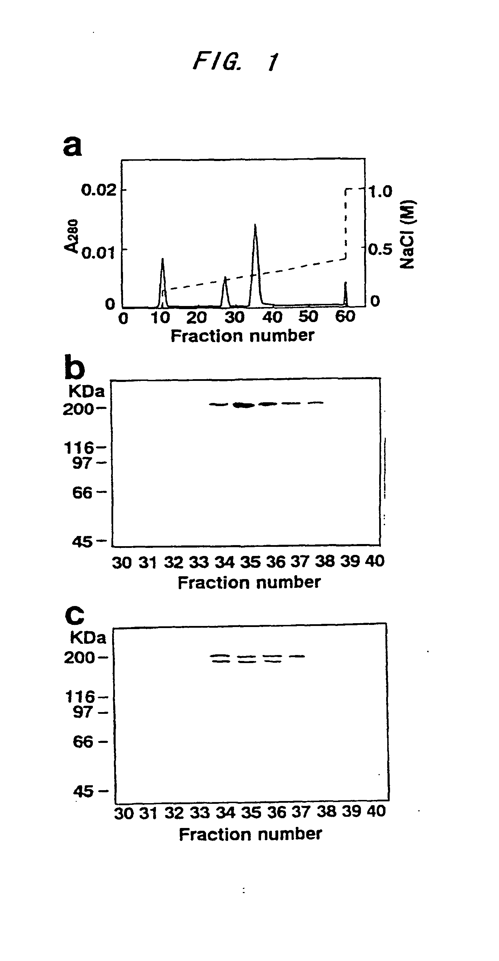

Next, the soluble fraction of rat fetal brain was subjected to SDS-PAGE and the protein band with a molecular weight (Mr) of 205 kDa was purified by column chromtographies such as Q-Sepharose, phenyl-5PW, hydroxyapatite, Mono Q. The result of the final Mono Q column chromatography is shown in Figure 1. In Figure 1, (a) shows the absorption at 280 nm, (b) shows the result of blot overlay with ¹²⁵I-labelled F-actin and (c) shows the protein bands stained with Coomassie brilliant blue. As shown in Figure 1 (c), the purified protein finally gave bands with a Mr of about 205 kDa (p205) and of about 190 kDa (p190) . Then, the two purified proteins were excised from the polyacrylamide gel, subjected to limited digestion with a protease (lysyl endopeptidase) and subjected to peptide mapping. Five peptides common to the two proteins were isolated and partial amino acid sequences thereof were separately determined. As a result of a homology search using a sequence data base, it was confirmed that the five peptide peaks were significantly homologous to those of human AF-6 protein. On the other hand, the amino acid sequence of the two peptide peaks specific to p205 were not found in current protein data base. The results suggested that p205 and p190 were human AF-6 protein-related rat proteins, and p190 was a splicing variant, a homologue, or a degradative product of p205. In addition, since the p205 was localized in the AJ site, the protein was named "a large splicing variant of AF-6 protein localized at adherens junction: l-Afadin" (hereinafter, the protein of the present invention is referred to as 1-Afadin or p205).

### Example 2: Cloning of a gene for the actin filament -binding protein "1-Afadin"

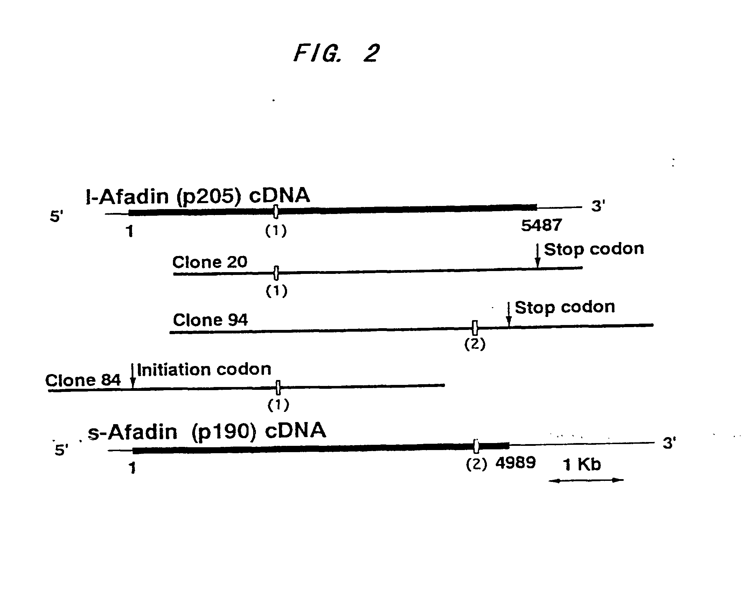

Based on the partial amino acid sequences of the 205 kDa protein l-Afadin obtained in Example 1, 7 oligonucleotide probes were prepared and used for screening a rat brain cDNA library. As a result, several overlapping clones as shown in Figure 2 were obtained. The result of sequencing indicated that, among these clones, clone 20 contained a coding region with about 4.9 kbp and the amino acid sequence deduced from this coding region included the whole peptides of p205. In addition, 2 peptides specific to p205 were localized in the C-terminal. Clone 94 contained a coding region of about 4.5kbp encoding p190. However, these clones 20 and 94 did not contain the initiation codon, which was contained in clone 84. Therefore, the full-length cDNA for p205 was constructed from clones 84 and 20, and the full-length cDNA for p190 from clones 84 and 94.

FISH analysis (Cytogenet. Cell Genet. 61:282-285,1992; Electrophoresis 16:261-272, 1995) using the clones 20, 84 and 94 as probes indicated that these cDNAs were localized on rat chromosome lq12.2.

### Example 3: Expression of F-actin-binding protein 1-Afadin in animal cells

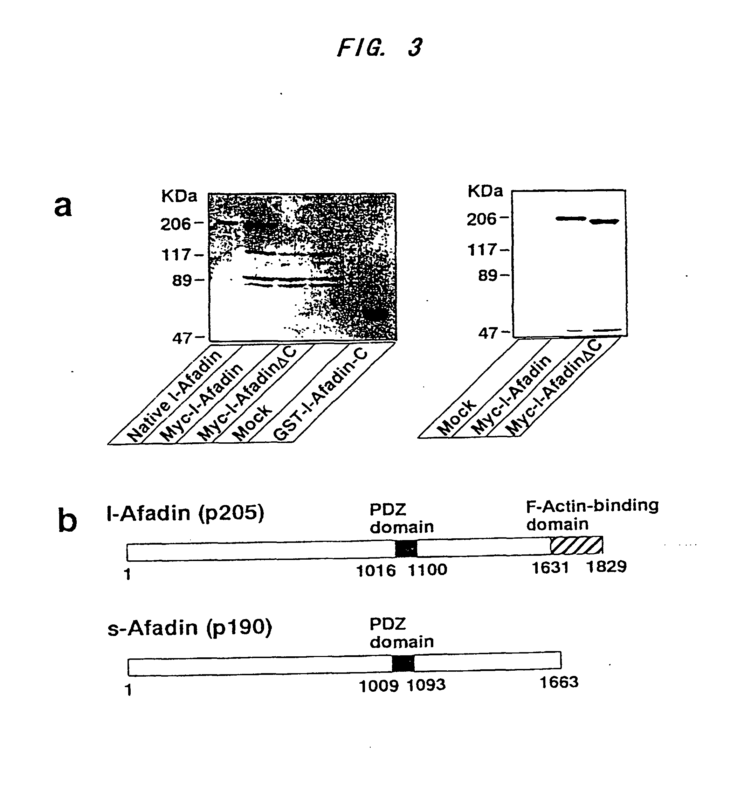

The p205 cDNA prepared in Example 2 was inserted into an expression vector, and transfected into COSl cells. The cell extract was subjected to the blot overlay method with ¹²⁵I-labelled F-actin. The recombinant protein (myc-l-afadin) showed a mobility on SDS-PAGE and binding activity to ¹²⁵1-labelled F-actin similar to that of native p205, as shown in Figure 3(a). On the other hand, the deletion mutant of p205 lacking the C-terminal 156 amino acid did not show the F-actin-binding activity. In contrast, a fusion protein of the C-terminal (199 amino acid residues) of p205 and GTS (glutathione-S-transferase) did show the ¹²⁵I-labelled F-actin-binding activity.

From the above results, it was confirmed that the p205 gene encodes a protein of 1,829 amino acids as showed in SEQ ID NO: 1, had an estimated molecular weight of 207,667 and had an F-actin-binding domain on 199 amino acid residues in the C-terminal. Further, it was concluded that the p190 gene encodes a protein lacking about 160 amino acid residues in the C-terminal and was a splicing variant of the p205 gene.

A computer homology search revealed that the amino acid sequence of p190 showed 90% identity over the entire sequence of human AF-6 protein. However, human AF-6 protein and p-190 lacked the C-terminal region of p205. Further, the C-terminal F-actin-binding domain showed no significant homology to any other F-actin-binding protein. Therefore, it was confirmed that, while p190 is likely to be a rat counterpart of human AF-6, p205 is a novel F-actin-binding protein. As shown in Figure 3(b), both p205 and pl90 had one PDZ domain.

### Example 4: Preparation of anti-1-Afadin antibody

According to known methods and using a synthetic peptide corresponding to amino acid residues 1814-1829 of SEQ ID NO:1 as an immunogen, a rabbit polyclonal antibody specifically recognizing l-Afadin was prepared. Also, using a synthetic peptide corresponding to amino acid residues 557-592 of SEQ ID NO: 1 as an immunogen, a rabbit polyclonal antibody specifically recognizing both l-Afadin and S-Afadin was prepared.

### Example 5: Confirmation of tissues expressing l-Afadin

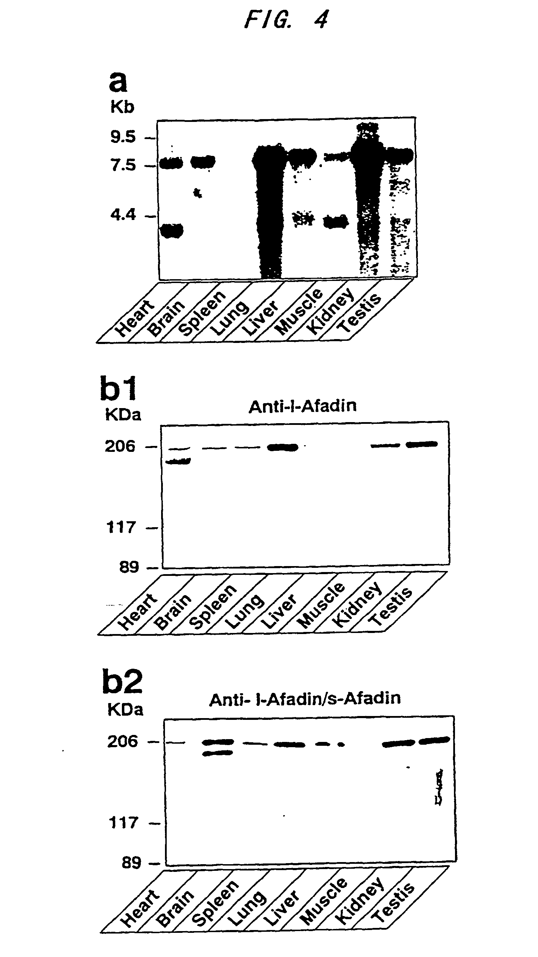

Northern blot analysis using a sequence specific to l-Afadin cDNA as a probe indicated that l-Afadin was ubiquitously expressed in all the rat tissues examined, including heart, brain, spleen, lung, liver, skeletal muscle, kidney and testis, as shown in Figure 4(a).

Further, it was confirmed, by Western blot analysis using the anti-l-Afadin antibody prepared in Example 4, that 1-Afadin was expressed in all the rat tissues, as shown in Figure 4 (bl). However, as shown in Figure 4 (b2), it was confirmed by Western blot analysis using the antibody recognizing both l-Afadin and s-Afadin that of all the organs tested, s-Afadin was only expressed in brain.

From the above results, it was confirmed that, while s-Afadin was expressed only in brain, l-Afadin of the present invention was ubiquitously expressed.

### Example 6: Biochemical characteristic of l-Afadin

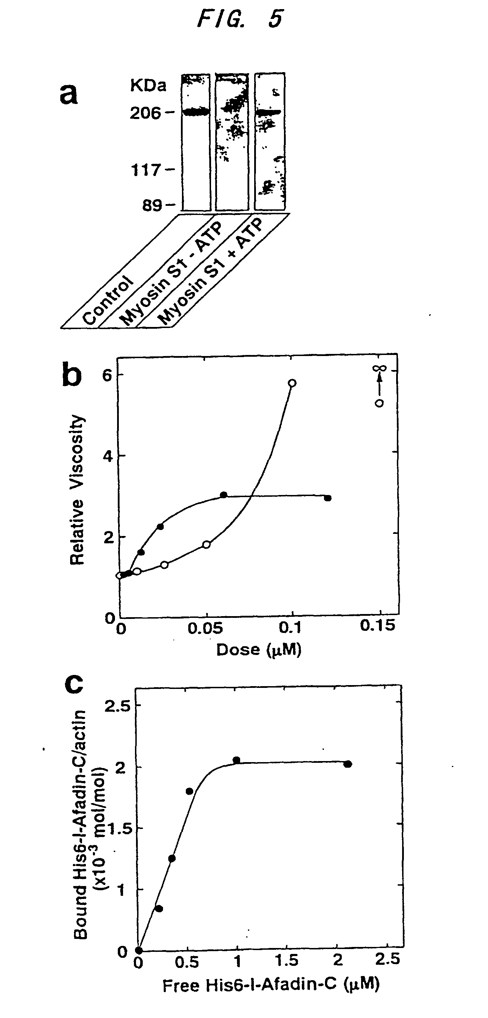

The blot overlay study for the actin-binding ability of the 205 kDa protein (l-Afadin) obtained in Example 1 revealed that the binding of l-Afadin to F-actin was specifically inhibited by myosin S1 (Figure 5(a)), but the inhibition disappeared by the addition of Mg ATP. Since myosin S1 is a protein which is confirmed as binding laterally to F-actin (Science 261:58-65, 1993; Nature 364:171-174, 1993) and Mg ATP is known to dissociate F-actin-myosin complex (Biochemistry 14:2207-2214, 1975), it was confirmed that l-Afadin binds along the side of F-actin.

Next, a change in viscosity of F-actin by l-Afadin was studied by the falling ball method (Methods Enzymol. 85:211-233, 1982; J. Biol. Chem. 271:31775-31778, 1996). It was found that, l-Afadin increased dose-dependently the viscosity of F-actin, up to a viscosity of about 3 times the maximum, as shown in Figure 5(b).

In addition, by stoichiometric calculation it was determined that His6-l-Afadin-C binds to F-actin at a ratio of 1 molecule per about 500 molecules of F-actin and that the Kd value was in a order of 10⁻⁷M (molar) (Figure 5(c)).

Further, the effect of 1-Afadin on F-actin was examined using pyrene-conjugated actin. It was found that 1-Afadin does not affect the actin polymerisation.

### Example 7: Localization of 1-Afadin

Using the anti-l-Afadin antibody, frozen slices of various mouse and rat tissues were observed with confocal microscopy to identify the localization of l-Afadin.

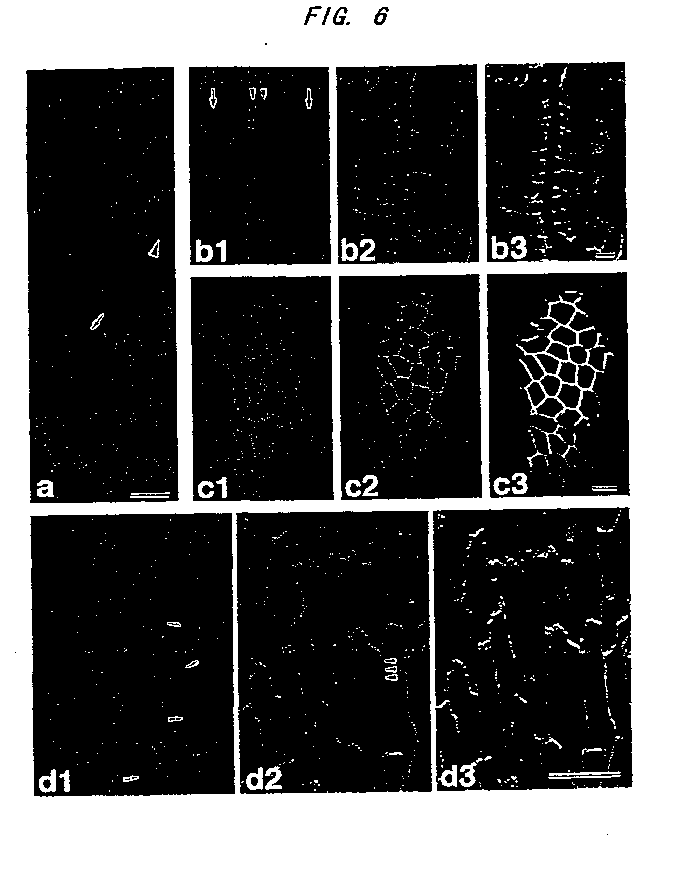

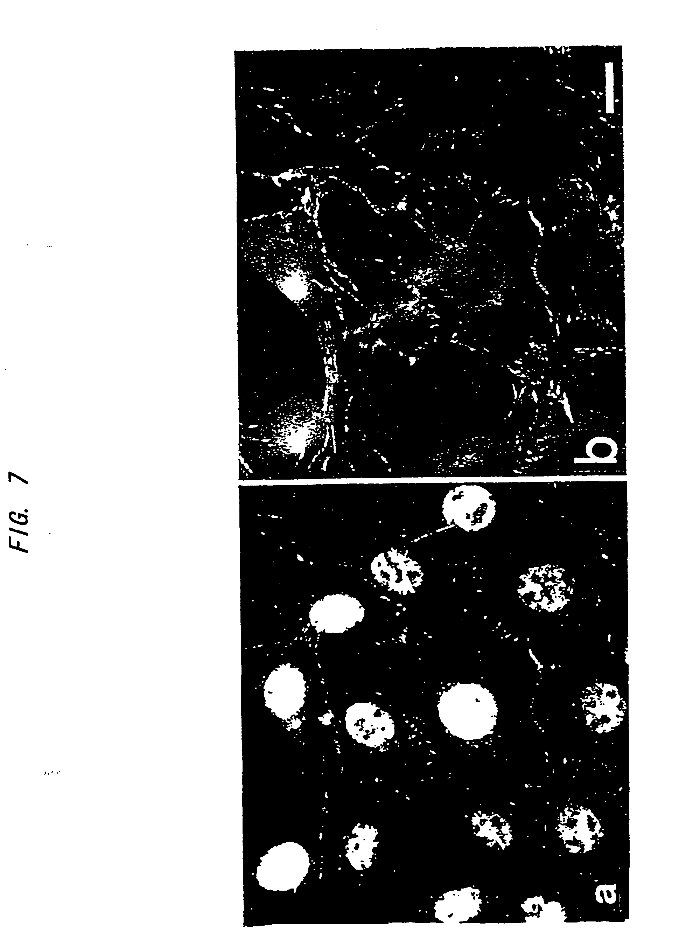

In liver, l-Afadin was localized in a belt-like junctional complex region along the bile canaliculi (Figure 6(a)). In the small intestine, which was doubly stained with the anti-E-cadherin monoclonal antibody, L-Afadin was detected in a junctional complex region of intestinal absorptive epithelium together with E-cadherin, but was more concentrated in the region than E-cadherin was (Figure 6(bl) - (b3) and (cl) - (c3)). Heart was doubly stained with the anti-vinculin monoclonal antibody. Vinculin is been known as a marker for not only cell-to-cell AJ but also for cell-to-matrix AJ (Cell 18:193-205, 1979; Biochem. Biophys. Acta 737:305-341, 1983). l-Afadin was found to co-localize with vinculin at intercalated discs (Figure 6(dl) - (d3)). However, while vinculin was also periodically located along the lateral border of cardiac muscle cells, l-Afadin was not detected in this region. In addition, when cultured EL cells expressing E-cadherin (Nature 329:341-343, 1987) were doubly stained with the anti-ZO-1 antibody, the localization of 1-Afadin was similar to that of ZO-1 (Figure 7(a) and (b)). Since ZO-1 is known to be concentrated at cadherin-based spot-like cell-to-cell AJ in fibroblast (J. Cell Biol. 115:1149-1462, 1991; J. Cell Biol. 121:491-502, 1993), it was suggested that 1-Afadin is also localized at cadherin-based cell-to-cell AJ.

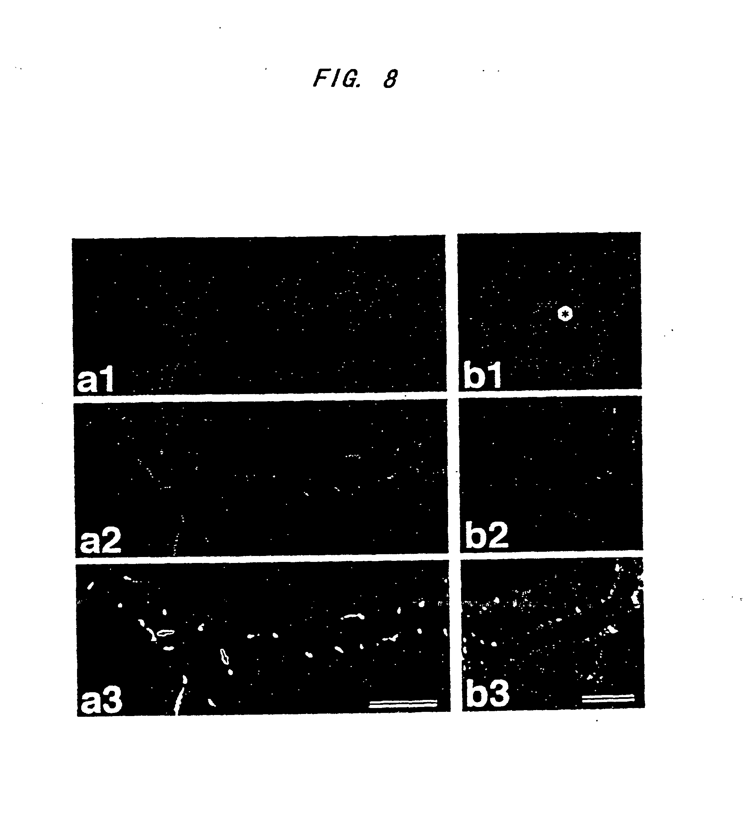

Further, in order to examine the precise localization of 1-Afadin, frozen sections of small intestine were doubly stained with the anti-ZO-1 monoclonal antibody and the anti-l-Afadin antibody. In addition, liver bile canaliculi were doubly stained with the anti-desmoplakin monoclonal antibody and the anti-l-Afadin antibody. ZO-1 is known to be a marker for tight junctions in intestinal absorptive epithelium (J. Cell Biol. 103:755-766, 1986; J. Cell Biol. 121:491-502, 1993) and desmoplakin is known to be a marker for desmosomes (J. Cell Biol. 63:515-523, 1974; Eur. J. Cell Biol. 32:117-130, 1983; J. Mol. Biol. 163:647-671, 1983; EMBO J. 6:885-889, 1987). The results showed that, in the absorptive epithelia of small intestine, 1-Afadin was localized slightly more it the basal side than ZO-1 (Figure 8(al) - (a3)). In the bile duct, the localisation of l-Afadin did not coincide with that of desmoplakin (Figure 8(b1)-(b3)).

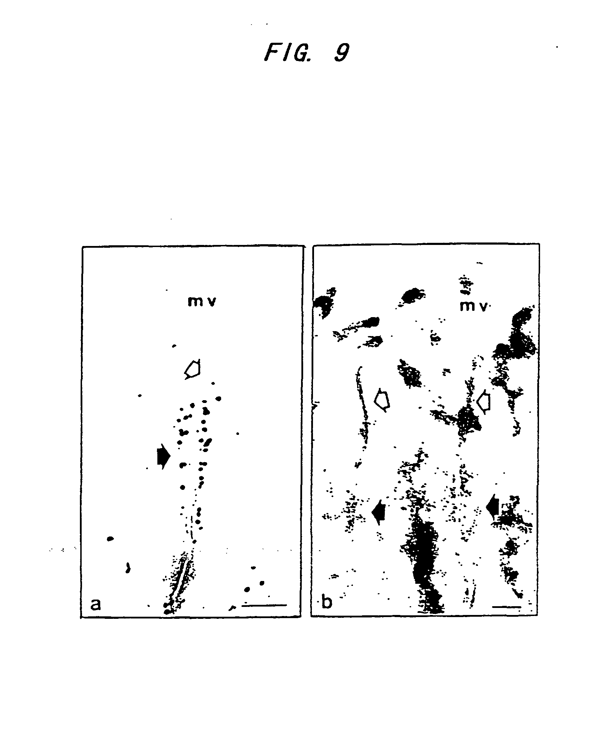

These results indicate that 1-Afadin is localized at cell-to-cell AJ rather than at tight junctions or desmosomes. Further, according to immunoelectron microscopy, it was observed that l-Afadin was localized in cell-to-cell AJ of absorptive epithelia of small intestine (Figure 9(a) and (b)).

Accordingly, it was confirmed that l-Afadin of the present invention is a novel protein uniting the actin cytoskeleton and cell-to-cell AJ.

As described above in detail, the present invention provides a novel actin filament-binding protein l-Afadin localized at the cadherin cell-to-cell adherens junction and the genetic materials for industrially utilizing 1-Afadin.

## Claims

1. An actin-binding protein 1-Afadin having the amino acid sequence of SEQ ID NO: 1.

2. A nucleic acid molecule comprising a nucleotide sequence encoding the protein as defined in claim 1.

3. A nucleic acid molecule as claimed in claim 2 wherein said nucleic acid molecule is cDNA.

4. A cloning or expression vector containing a nucleic acid molecule as defined in claim 2 or 3.

5. A host cell transformed with a nucleic acid molecule as defined in claim 2 or 3 or containing a nucleic acid molecule as defined in claim 3.

6. A method of preparing a protein, as defined in claim 1, wherein said method comprises culturing a host cell containing a nucleic acid molecule encoding said protein, under conditions whereby said protein is expressed and recovering said protein thus produced.

7. A synthetic protein as defined in claim 1 expressed by a host cell as defined in claim 5.

## Patentansprüche

1. Actin-bindendes Protein 1-Afadin mit der Aminosäuresequenz SEQ ID NO:1.

2. Nukleinsäuremolekül, das eine das in Anspruch 1 definierte Protein kodierende Nukleotidsequenz umfasst.

3. Nukleinsäuremolekül nach Anspruch 2, wobei das Nukleinsäuremolekül cDNA ist.

4. Klonierungs- oder Expressionsvektor, der ein in Anspruch 2 oder 3 definiertes Nukleinsäuremolekül enthält.

5. Wirtszelle, die mit einem in Anspruch 2 oder 3 definierten Nukleinsäuremolekül transformiert ist oder ein in Anspruch 3 definiertes Nukleinsäuremolekül enthält.

6. Verfahren zur Herstellung eines in Anspruch 1 definierten Proteins, wobei man eine Wirtszelle, die ein das Protein kodierendes Nukleinsäuremolekül enthält, unter Bedingungen kultiviert, wodurch das Protein exprimiert wird, und das auf diese Weise produzierte Protein gewinnt.

7. In Anspruch 1 definiertes synthetisches Protein, das von einer in Anspruch 5 definierten Wirtszelle exprimiert ist.

## Revendications

1. Protéine 1-afadine qui se lie à l'actine, possédant la séquence d'acides aminés de la SEQ ID n°1.

2. Molécule d'acide nucléique comprenant une séquence nucléotidique codant pour la protéine telle que définie à la revendication 1.

3. Molécule d'acide nucléique selon la revendication 2 où ladite molécule d'acide nucléique est une molécule d'ADNc.

4. Vecteur de clonage ou d'expression contenant une molécule d'acide nucléique telle que définie à la revendication 2 ou 3.

5. Cellule hôte transformée avec une molécule d'acide nucléique telle que définie à la revendication 2 ou 3 ou contenant une molécule d'acide nucléique telle que définie à la revendication 3.

6. Procédé de préparation d'une protéine telle que définie à la revendication 1, où ledit procédé comprend la culture d'une cellule hôte contenant une molécule d'acide nucléique codant pour ladite protéine, dans des conditions dans lesquelles ladite protéine est exprimée et la récupération de ladite protéine ainsi produite.

7. Protéine synthétique telle que définie à la revendication 1 exprimée par une cellule hôte telle que définie à la revendication 5.
